# EUROPEAN PATENT SPECIFICATION

(11) **EP 0 237 767 B1**
(45) Date of publication and mention of the grant of the patent: **01.09.1993**
(21) Application number: 87101814.9
(22) Date of filing: 10.02.1987
(51) Int. Cl.: A61N 1/365, A61N 1/368

(54) **Rate responsive pacing using the ventricular gradient**
Taktempfindliche Stimulierung mit Benutzung des ventrikulären Gradienten
Stimulation sensible au rythme, utilisant le gradient ventriculaire

(30) Priority: 19.03.1986 US 841478; 19.03.1986 US 841305; 19.03.1986 US 841517
(43) Date of publication of application: 23.09.1987
(73) Proprietor: TELECTRONICS N.V., Willemstad Curaçao (AN)
(72) Inventor: Callaghan,Frank J., Miami,Florida 33186 (US)
(74) Representative: KUHNEN, WACKER & PARTNER

(56) References cited:
- EP-A- 0 007 189
- EP-A- 0 017 848
- EP-A- 0 232 528
- GB-A- 2 098 489
- US-A- 4 313 442

## Description

### BACKGROUND OF THE INVENTION

An implantable cardiac pacer can have various pacing modes as well as various output parameters such as rate, output level (current, voltage, pulse width sensitivity, refractory period, etc. In some cardiac pacers both the mode, e.g, R-wave inhibited VVI, as well as the various output parameters, are preset during production, whereas in other cardiac pacers either mode or output parameters or both can be altered by external control or programming. Such output parameters and/or pacing mode changes are usually accomplished by the attending physician, usually during an office visit. Therefore, such cardiac pacers may not be responsive to the physiological requirements of the patients. Such requirements fluctuate often during a 24-hour period, certainly more frequently than the interval between patient's visits to the physician. Thus the patient must suffer less than optimum heart pacing.

Increased emphasis is being placed on the use of physiological parameters to control the output parameters, and particularly the rate of stimulation, of implantable cardiac pacers. Such physiological parameters can include activity of the patient (Dahl U.S. Patent No. 4,140,132), sensed ionic changes (Wirtzfeld U.S. Patent No. 4,202,339), the patient's threshold, i.e., the level of a stimulus pulse required to evoke a resulting heartbeat when the pulse is delivered to the patient's heart (Wittkampf, et al. U.S. Patent No. 4,305,396), and the stimulus to repolarization of the T-wave interval (Rickards U.S. Patent No. 4,228,803). The detection of such changes in utilized either to increase or to decrease the rate of stimulation.

Measurement of physical activity or of the ionic level in the blood does not appear to measure the effectiveness of the pulse emitted from the cardiac pacer initiating myocardial contraction.

While the stimulus to repolarization interval on the T-wave can provide information as to stress on the heart, the T-wave is sometimes difficult to detect. This is illustrated in the article by Fananapazir entitled Reliability of the Evoked Response in Determining the Paced Ventricular Rate and Performance of the QT of Rate Responsive (TX) Pacemaker, "Pace", vol. 8, pp. 701-714 (September-October, 1985). In this article, it is stated that problems resulted from attempts to provide T-wave sensing in the long term with patients where the system was being used to control heart pacing rates.

Also the amplitude, as measured from the base line, of the repolarization potential or T-wave tends to be low, thereby posing problems in detection. In addition, within means for detecting the evoked potential of the T-wave, a time period or "window" for sensing of the T-wave must be established and programmed either at manufacture or after implantation. If there is an improper setting of the sensing window, the T-wave may not be sensed. Also, in certain patients, the T-wave may be bimodal; that is, there may be two peaks rather than one. It is possible that the amplitude of the first peak is not sufficient to be detected, and that the second peak may be outside of the sensing window. Alternatively, neither of the peaks of the bimodal T-wave may be of sufficient amplitude to be detected.

EP-A-0 232 528, a document under Art. 54 (3) EPC, discloses a cardiac pacing apparatus which comprises:
means for applying electrical stimulus pulses to the heart;
means for detecting a cardiac event potential;
means for integrating the detected potential over time to obtain the depolarization gradient duration as a selected parameter;
means for storing said selected parameter;
means for comparing said selected parameter with a corresponding selected parameter of at least one previous cardiac cycle that has been stored; and
means for controlling the rate of said electrical stimulus pulses in response to said comparison.

Furthermore, EP-A-0 017 848 discribes a rate adaptive pacemaker wherein the rate of delivered stimulus pulses is controlled as a function of sensed physiological changes, such as T-wave sensing.

US-A-4 313 442 discloses an atrial rate sensitive demand pacer which is supplied with means for detecting changes in the fundamental period of the atrial electrical cycle, averageing these changes of a predetermined time interval and using the resulting control signal to control the pacer.

GB-A-2 098 489 discloses a signal processing apparatus for processing a sensed signal from a muscle of a mammalian body in order to produce a control signal which can be refed into the muscle and, which has a characteristic proportional to the amplitude of the sensed signal.

In addition to this, EP-A-0 007 189 describes a physiologically adaptive pacer which is designed for sensing the presence or the absence of an evoked T-wave following a delivered stimulus pulse.

In accordance with this invention, a more reliable technique for detecting stress level changes is provided, particularly in a paced heart where it is desirable to provide feedback means so that the rate of pacing of the heart can vary in a manner responsive to heart stress. Improvements of long term reliability and ease of operation are provided by this invention.

### SUMMARY OF THE INVENTION

In this invention, a cardiac pacing apparatus is provided for applying electrical stimulus pulses to the heart in a manner that is at least partially controlled by stress level changes. Electrical stimulus pulses are applied to pace the heart at a rate which is at least partially dependent on the stress level changes so determined, to provide a heart pacing system which is responsive in terms of causing a changed heartbeat rate in response to changes in stress levels of the heart. This, in turn, provides greater patient comfort and greater ability for the patient to lead a normal life, engaging in a greater range of activities calling for different pulse rates.

In a first embodiment of this invention, a cardiac pacing apparatus is provided which comprises:
means for applying electrical stimulus pulses to the heart ;
means for detecting a cardiac event potential;
means for integrating the detected potential during at least one cardiac cycle to obtain the depolarization gradient magnitude as a selected parameter;
means for storing said selected parameter;
means for comparing said selected parameter with a corresponding selected parameter of at least one previous cardiac cycle that has been stored; and
means for controlling the rate of said electrical stimulus pulses in response to said comparison.

With such a pacer, a system is provided for detecting in at least one cardiac cycle, the changing voltage of the cardiac ventricle during the QRS phase of the cycle; followed by integrating the changing voltage over time from essentially the Q-point to the S-point to obtain the depolarization gradient. The system then compares the depolarization gradient magnitude of one cardiac cycle (i.e., heartbeat) with the depolarization gradient magnitude of at least one previous cardiac cycle. A change in the value of the depolarization gradient magnitude is an indication of a change in heartbeat stress level. The changes are inversely dependent upon stress in the heart: an increase in the depolarization gradient magnitude indicates a decrease in heart stress, while a decrease in the depolarization gradient magnitude indicates an increase in heart stress. Accordingly, when the depolarization gradient magnitude is sensed to decrease in a pacing system, means are provided to increase the pacing rate to increase the heartbeat and heart output. Conversely, when the depolarization gradient magnitude increases, it is an indication of less stress and the pacing system slows the heartbeat in response. When the depolarization gradient magnitude remains the same, it is an indication that there is no change in stress.

Additionally, the system may compare parameters of the QRS complex which indicate heart stress against a target value which has been determined by the medical history of the patient. For example, the system may determine the depolarization gradient magnitude of at least one heartbeat and then compare the value of the depolarization gradient magnitude obtained with such target value. If the depolarization gradient magnitude is less than the target value, the system may increase the rate of electrical stimuli emitted by the heart pacer. If the depolarization gradient magnitude is greater than the target value, system may decrease the rate of such electrical stimuli.

In a second embodiment of this invention, a cardiac pacing apparatus is provided which comprises:
means for applying electrical stimulus pulses to the heart ;
means for detecting a cardiac event potential;
means for integrating the detected potential during at least one cardiac cycle to obtain the repolarization gradient as a selected parameter;
means for sensing the peak of said repolarization gradient ;
means for determining the repolarization gradient magnitude;
means for storing said repolarization gradient magnitude;
means for comparing said repolarization gradient magnitude with a corresponding selected parameter of at least one previous cardiac cycle that has been stored;
and
means for controlling the rate of said electrical stimulus pulses in response to said comparison.

Therefore, in accordance with the present invention, a system is provided for detecting in at least one cardiac cycle, the changing voltage of the cardiac ventricle during the QRST phase of the cycle; followed by integrating the changing voltage over time from essentially the Q-point to the T-point to obtain the repolarization gradient. The system then compares the repolarization gradient magnitude of one cardiac cycle (i.e., heartbeat) with the repolarization gradient magnitude of at least one previous cardiac cycle. A change in the value of the repolarization gradient magnitude is an indication of a change in heartbeat stress level. The changes are dependent upon stress in the heart: an increase in the repolarization gradient magnitude indicates an increase in heart stress, while a decrease in the repolarization gradient magnitude indicates a decrease in heart stress. Accordingly, when the repolarization gradient magnitude is sensed to increase in a pacing system, means are provided to increase the pacing rate to increase the heartbeat and heart output. Conversely, when the repolarization gradient magnitude decreases, it is an indication of less stress and the pacing system slows the heartbeat in response. When the repolarization gradient magnitude remains the same, it is an indication that there is no change in stress.

Additionally, the system may compare parameters of the QRST complex which indicate heart stress against a target value which has been determined by the medical history of the patient. For example, the system may determine the repolarization gradient magnitude of at least one heartbeat and then compare the value of the repolarization gradient magnitude obtained with such target value. If the repolarization gradient magnitude is greater than the target value, the system may increase the rate of electrical stimuli emitted by the heart pacer. If the repolarization gradient magnitude is less than the target value, the system may decrease the rate of such electrical stimuli.

In a third embodiment of this invention, a system is provided for detecting in at least one cardiac cycle, the changing voltage of the cardiac ventricle during the QRST phase of the cycle; followed by integrating the changing voltage over time from essentially the Q-point to the S-point to obtain the depolarization gradient, and from the Q-point to the T-point to obtain the repolarization gradient. The system then determines the peak to peak time interval from the peak of the depolarization gradient to the peak of the repolarization gradient. The peak to peak time interval of one cardiac cycle (i.e., heartbeat) is compared with the peak to peak time interval of at least one previous cardiac cycle. A change in the value of the peak to peak time interval is an indication of a change in heartbeat stress level. The changes are inversely dependent upon stress in the heart: an increase in the peak to peak time interval indicates a decrease in heart stress, while a decrease in the peak to peak time interval indicates an increase in heart stress. Accordingly, when the peak to peak time interval is sensed to increase in a pacing system, means are provided to decrease the pacing rate to decrease the heartbeat and heart output. Conversely, when the peak to peak time interval decreases, it is an indication of more stress and the pacing system increases the heartbeat in response. When the peak to peak time interval remains the same, it is an indication that there is no change in stress.

Additionally, the system may compare parameters of the QRS complex which indicate heart stress against a target value which has been determined by the medical history of the patient. For example, the system may determine the peak to peak time interval of at least one heartbeat and then compare the peak to peak time interval obtained with such target value. If the peak to peak time interval is greater than the target value, the system may decrease the rate of electrical stimuli admitted by the heart pacer. If the peak to peak time interval is less than the target value, the system may increase the rate of such electrical stimuli.

As a result of this, a patient having a paced heart is no longer limited to a single electric stimulus rate from visit to visit to the doctor's office to adjust such rate. If he climbs a hill or a flight of stairs, his pacer stimulus rate will increase. When he goes to bed, his stimulus rate will decrease. Thus, patients who are equipped with pacers controlled in accordance with this invention can have a lifestyle which is much closer to normal than in previous pacer systems.

One significant advantage of the ventricular gradient is that its value is independent of the origin of heart stimulation. Hence, naturally conducted beats, paced beats, and ectopic beats can all yield heart stress information by the comparison of ventricular gradients of a present heartbeat with an earlier heartbeat in accordance with this invention.

It should be noted that sensing of the changing voltage of the heart ventricle provides more meaningful data than sensing voltage changes of other portions of the heart, although the system may also sense in accordance with this invention from the atrium.

### BRIEF DESCRIPTION OF THE DRAWINGS

FIGURE 1 is an elevational view of a cardiac pacing system with a bipolar lead which functions both as a unipolar and a bipolar system at different steps of the operating cycle;
FIGURE 2 is a timing diagram of the relationship of the electronic events which take place during a single cardiac cycle;
FIGURE 3 is a schematic block diagram of a single chamber cardiac pacer with evoked potential monitoring and an integrating circuit;
FIGURE 4 is a schematic block diagram of the charge dump circuit used herein;
FIGURE 5 is a diagram of the evoked potential over time sensed by a pacer and lead of FIGURE 1 positioned at a heart ventricle;
FIGURE 6 is a diagram showing the integrated value of the evoked potential of FIGURE 5, in horizontal time synchronization with FIGURE 5;
FIGURE 7 is a schematic diagram of a pacer system constructed in accordance with the present invention;
FIGURES 8A and 8B, when connected together, comprise a flow chart depicting the operation of the diagram of FIGURE 7 for one embodiment of the invention;
FIGURES 9A and 9B, when connected together, comprise a flow chart depicting the operation of the diagram of FIGURE 7 for a second embodiment of the invention; and
FIGURES 10A and 10B, when connected together, comprise a flow chart depicting the operation of the diagram of FIGURE 7 for a third embodiment of the invention.

### DETAILED DESCRIPTION OF THE ILLUSTRATIVE EMBODIMENTS

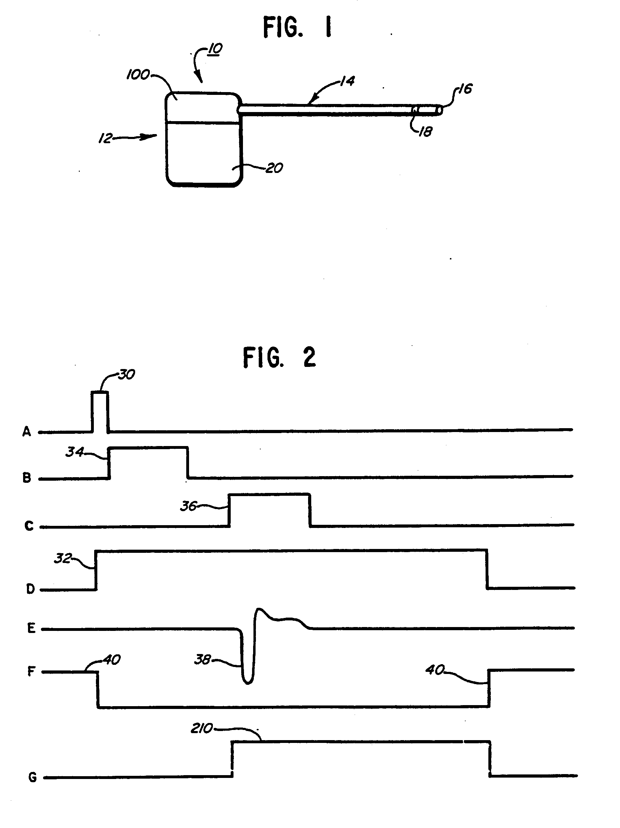

Referring to the drawings, in FIGURE 1 a single chamber cardiac pacing system 10 includes pulse generator 12, which may be of generally conventional electronics except as otherwise described herein. Pervenous bipolar lead 14 is also provided and may be of conventional bipolar pervenous or epicardial design.

First electrode 16 may be a porous, platinum-iridium, hemispherically shaped electrode on the distal end of lead 14, communicating with a metal conductor inside of the lead. Ring electrode 18 serves as a second electrode, being capable of electrical isolation by means of a circuitry and pulse generator 12 from first electrode 16, and being typically spaced at least 0.5 cm. from first electrode 16. Second, ring-shaped electrode 18 may also be made of the same alloy and may communicate with its own circuit wire within lead 14 and may or may not be porous-coated.

The circuitry of pulse generator 12 may be sealed in a hermetic container, for example, titanium can 20, as shown. When the pacer can 20 is treated as an independent electrode, the single chamber cardiac pacing system 10 carries three electrodes: can 20, first electrode 16 and second electrode 18. The operation of the pacing system as described applies to both the atrial and ventricular leads of a dual chamber cardiac pacer or an atrial standby pacer. However, for purposes of simplicity of disclosure, the details of operation will be disclosed for only a ventricular pacer. For additional disclosure concerning the detection of cardiac evoked potentials, reference is made to Herscovici U.S. Patent No. 4,543,956 and to the evoked potential detection and pacing system disclosed in copending U.S. application Serial No. 807,547, filed December 11, 1985, in the names of Frank J. Callaghan and Edward A. Schroeppel, and entitled "Detection Of Cardiac Evoked Potentials."

Cardiac pacing system 10 may be external or surgically installed into the patient, and may operate to pace the patient's heart as follows:

A pacing cycle begins when an electrical stimulus is emitted from first electrode 16 to stimulate muscular contraction of at least a portion of the heart. The stimulus is of a magnitude and width which is not harmful to the heart and which is well-known to those skilled in the art to evoke a contraction response from the heart muscle. The pulse of electric stimulus 30 is graphed in FIGURE 2 at channel A, having a typical duration of 0.1 to 2 milliseconds.

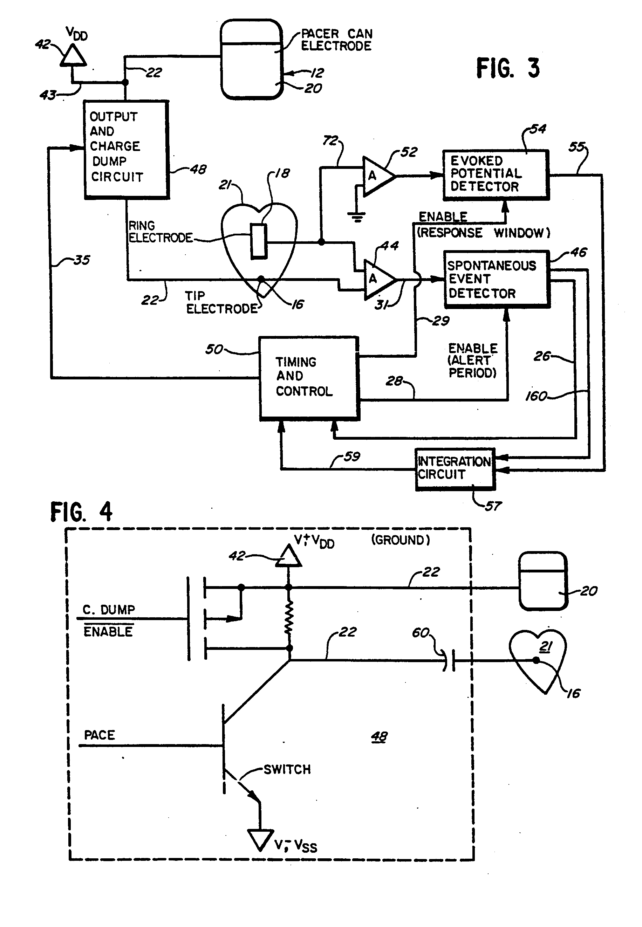

Referring to the circuit of FIGURE 3, pacer can 20 is shown serving as a reference electrode for electrodes 16, 18, carried at the heart 21 which is shown in schematic manner. Stimulus 30 is transmitted via conductor 22 to be emitted from tip electrode 16. The naturally occurring cardiac electrical activity is amplified by amplifier 44 and transmitted via line 31 to a spontaneous event detector 46 to begin a timing process. The signal proceeds via conductor 26 into timing and control circuitry module 50 which, in turn, has feedback and control wires 28, 29 connected, respectively, to detector 46 and to evoked response detector 54. Likewise, an output from timing and control circuit 50 is connected via line 35 to output and charge dump circuit 48.

Immediately following the emission of pulse 30 from electrode 16, charge dump circuit 48 is activated, with the charge dump pulse 34 being illustrated on channel B of FIGURE 2, the duration of the charge dump being about 5 to 15 milliseconds. The charge dump may be provided using a conventional charge dump circuit 48 such as illustrated in FIGURE 4. During the charge dump period, the electrical charge on output coupling capacitor 60 (FIGURE 4) and first electrode 16 are discharged through the heart 21. Thus the post-stimulus polarization potential of electrode 16 is quickly diminished.

Evoked response detector 54 is then activated by timing and control circuit 50 through conductor 29. A window of time 36 is opened as illustrated in channel C of FIGURE 2, its magnitude being typically 10 to 50 milliseconds. It is only during this time that evoked response detector 54 is activated to detect an evoked electrical response from the heart and to indicate a contractile response to the physiological pulse of electric potential 30.

The stimulus from electrode 16 can be seen to be in the unipolar mode. Likewise, detection of the evoked response is unipolar, being detected by electrode 18, which communicates through conductor 72 to amplifier 52, which sends the amplified signal to detector 54. Detector 54 transmits the detected signal via line 55 to integration circuit 57. The integrated signal, which is discussed in detail below, is transmitted to timing and control circuit 50 via line 59.

It is noted that the window of time 36 on channel C of FIGURE 2 is positioned in a block of time 32 (channel D of FIGURE 2) which generally represents a refractory period in which first electrode 16 may not be used to sense any electrical activity. However, the evoked response can be detected during a refractory period 32.

Channel E in FIGURE 2 shows the evoked cardiac electrical activity 38 within evoked response detection period 36, and which is detected by second electrode 18. The evoked heartbeat response 38 is detected by second electrode 18 in the unipolar mode. The detected evoked response which is fed via line 55 to integration circuit 57 and via line 59 to timing and control circuit 50 may serve to set the timer to zero for timing the next physiological pulse to be emitted from first electrode 16.

However, there is a need to determine that natural heartbeats will not show up at unexpected times, to avoid the result where the pacing system disrupts and interferes with the natural heartbeat. To this end, beginning essentially at the end of refractory period 32, during which spontaneous event detector 46 is disabled from sensing electric pulses, an alert period 40 (channel F; FIGURE 2) is provided to monitor a naturally occurring cardiac electrical activity until such time as the next pulse 30 is sent out through first electrode 16. Alert circuitry 46 may be activated and shut down by timing and control circuit 50 via line 28. When the alert phase 40 is in operation, both electrodes 16 and 18 are operating together in a bipolar mode, with both electrodes communicating with amplifier 44, which in turn is connected to spontaneous event detector 46.

In the event of a spontaneous heartbeat, a signal may be sent from spontaneous event detector 46 via line 26 to timing and control circuit 50, to cause the electronics to recycle from any time in the cycle to the beginning of the cycle, without generation of an electric pulse 30 from first electrode 16. Every time natural cardiac electrical activity takes place during alert period 40, no electric pulse 30 will be generated.

In the event, however, that detector 46 does not detect natural cardiac electrical activity during the alert period, timing and control circuit 50 will cause another electric pulse to be generated via electrode 16.

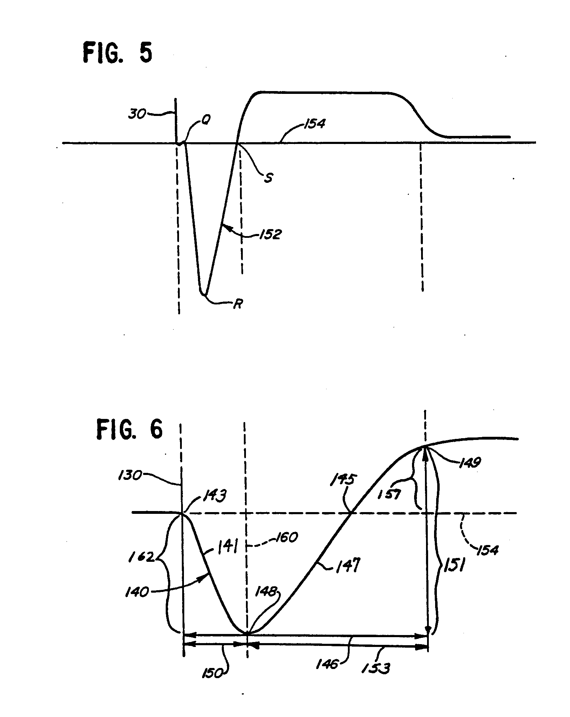

Referring to FIGURE 5, a typical cardiograph tracing of the changing potential in the ventricle of a heart is shown throughout most of a single cardiac cycle with respect to a reference base line of a predetermined voltage, typically zero volts. For purposes of this invention, the Q-point represents the beginning of the R-wave 152 where the voltage trace crosses or is closest to base line 154, prior to forming R-wave 152. The R-point is the peak of R-wave 152, irrespective of whether the trace is shown in its form of FIGURE 5 or in inverted form, which is possible with other recording systems. The S-point is where the trace crosses base line 154.

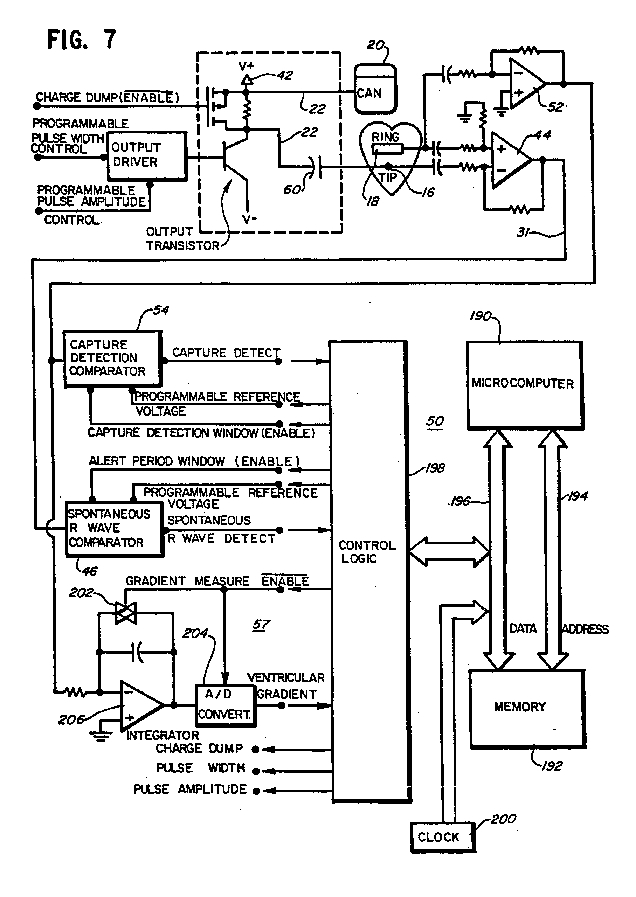

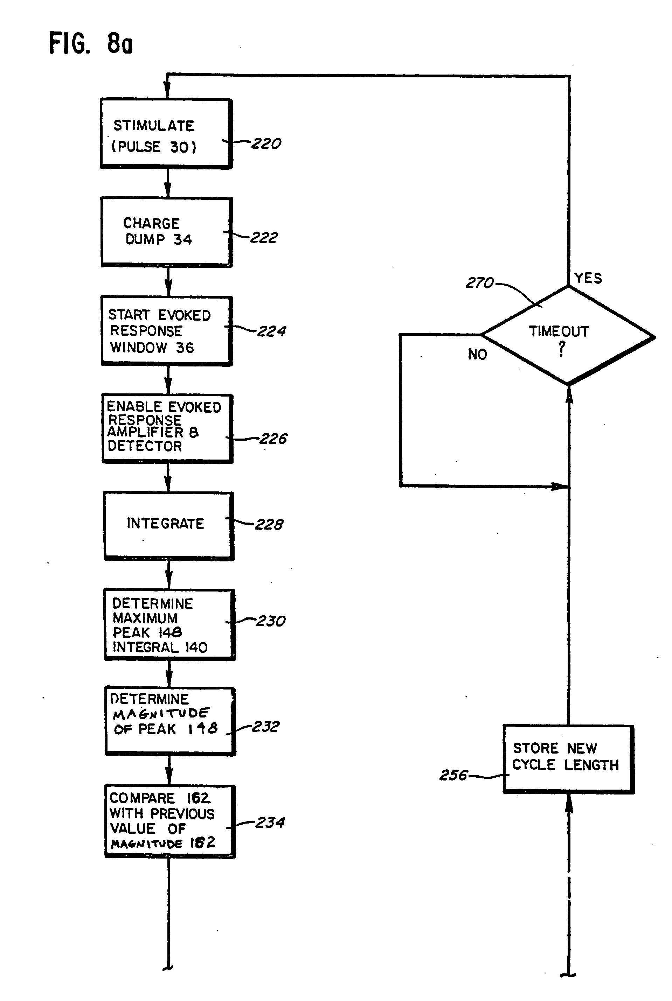

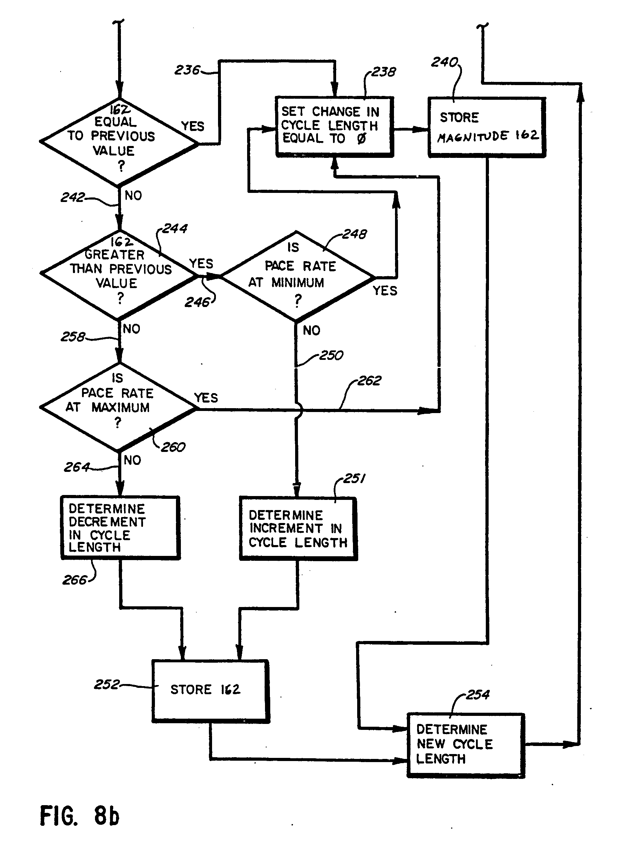

In operation, as illustrated in FIGURE 3, the evoked potential is detected on ring electrode 18. The signal is transmitted via heart amplifier 52 and detector 54 to integration circuit 57 via line 55. The integrated signal 140 is known as the ventricular gradient, and is illustrated in Figure 6.

Referring to Figure 6, the major parameter of interest to the first embodiment of the present invention is the magnitude 162 of the ventricular gradient 140, from the base line 154 to the peak 148. This magnitude may be referred to as the depolarization gradient magnitude 162.

During periods of heart stress, R wave 152 decreases in magnitude and the QS interval decreases in duration. Therefore, the depolarization gradient magnitude 162 will similarly decrease in magnitude. The depolarization magnitude 162 lends itself to detection and analysis. The occurrence of bimodal R waves will not negatively impact upon the value of the depolarization gradient magnitude 162 as a stress measuring tool.

The depolarization gradient magnitude 162 is calculated and compared to the depolarization gradient magnitude of a response detected prior to this one. The average time of three (or some predetermined number of) depolarization gradient magnitudes may be used. In this case, each response should change in the same direction. If the depolarization gradient magnitude 162 is equal to the previous value, there is no change in the heart pacing stimulus rate. The escape interval remains the same. If the depolarization gradient magnitude 162 is shorter than the prior value, and there has been a change in the same direction of the three (or some other predetermined number) depolarization gradient magnitudes, a determination is made as to whether or not the stimulus rate is at its programmed maximum rate. If it is at its maximum rate, the depolarization gradient magnitude is stored and the stimulus rate is not increased. However, if the stimulus rate is less than the programmed maximum rate, the rate is incremented by some predetermined value, and the depolarization gradient magnitude is stored for reference at the next subsequent integrated evoked heart response. Should the depolarization gradient magnitude increase, indicating a reduction in stress, the determination is made as to whether or not the rate of stimulation is at its minimum programmed rate. If it is at the programmed minimum rate, the depolarization gradient magnitude is stored with no decrease in rate. If it is not at its minimum programmed rate, the rate of the stimulation is decreased by some predetermined value, and the depolarization gradient magnitude is stored for future reference.

With respect to the second embodiment, the repolarization gradient is also of interest. The repolarization gradient includes portion 147 of th e waveform shown on Figure 6. The magnitude of the repolarization gradient is identified by reference numeral 151.

The repolarization gradient magnitude 151 (Figure 6) is the distance from the baseline 154 to point 149. This repolarization gradient magnitude 151 may be determined by subtracting the depolarization magnitude 162 from the peak 148 to peak 149 amplitude 157. Alternatively, the peak 148 to peak 149 amplitude 157 may be considered the repolarization gradient magnitude.

The repolarization gradient magnitude 151 is thus calculated and compared to the repolarization gradient magnitude of a response detected prior to this one. The average time of three (or some predetermined number of) repolarization gradient magnitudes may be used. In this case, each response should change in the same direction. If the repolarization gradient magnitude 151 is equal to the previous value, there is no change in the heart pacing stimulus rate. The escape interval remains the same. If the repolarization gradient magnitude 151 is shorter than the prior value and there has been a change in the same direction of the three (or some other predetermined number) repolarization gradient magnitudes, a determination is made as to whether or not the stimulus rate is at its programmed minimum rate. If it is at its minimum rate, the repolarization gradient magnitude is stored and the stimulus rate is not decreased. However, if the stimulus rate is more than the programmed minimum rate, the rate is decremented by some predetermined value, and the repolarization gradient magnitude is stored for reference at the next subsequent integrated evoked heart response. Should the repolarization gradient magnitude increase, indicating an increase in stress, the determination is made as to whether or not the rate of stimulation is at its maximum programmed rate. If it is at the programmed maximum rate, the repolarization gradient magnitude is stored with no increase in rate. If it is not at its maximum programmed rate, the rate of the stimulation is increased by some predetermined value, and the repolarization gradient magnitude is stored for future reference.

With respect to the third embodiment, the peak to peak time interval is also of interest. The time interval from the peak 148 of the depolarization gradient 141 to the peak 149 of the repolarization gradient 147 is identified by reference numeral 153.

The peak to peak time interval 153 is thus calculated and compared to the peak to peak time interval of a response detected prior to this one. The average time of three (or some predetermined number of) peak to peak time intervals may be used. In this case, each response should change in the same direction. If the peak to peak time interval 153 is equal to the previous value, there is no change in the heart pacing stimulus rate. The escape interval remains the same. If the peak to peak time interval 153 is shorter than the prior value and there has been a change in the same direction of the three (or some other predetermined number) peak to peak time intervals, a determination is made as to whether or not the stimulus rate is at its programmed maximum rate. If it is at its maximum rate, the peak to peak time interval is stored and the stimulus rate is not increased. However, if the stimulus rate is less than the programmed maximum rate, the rate is incremented by some predetermined value, and the peak to peak time interval is stored for reference at the next subsequent integrated evoked heart response.

Should the peak to peak time interval increase, indicating a reduction in stress, the determination is made as to whether or not the rate of stimulation is at its minimum programmed rate. If it is at the programmed minimum rate, the peak to peak time interval is stored with no decrease in rate. If it is not at its minimum programmed rate, the rate of the stimulation is decreased by some predetermined value, and the peak to peak time interval is stored for future reference.

Additionally, spontaneous electrical events such as those conducted from the atrium to the ventricle via the cardiac conduction pathway or those arising within the ventricle itself, and premature ventricular contractions are detected bipolarly by the ring and tip. These signals are amplified by the amplifier 44 and detected in the spontaneous event detector 46. The timing and control circuit 50 acts upon these events to reset the escape interval. Further, these spontaneous electrical events may be integrated if desired, and the magnitude 162 of the depolarization gradient may be determined. Rate changes or escape interval changes may be implemented based on the depolarization gradient magnitude of the spontaneous electrical events in the same manner that they are implemented based on the depolarization gradient magnitude of the evoked potentials. To this end, the integration circuit 57 of FIGURE 3 is shown as receiving the signal from the spontaneous event detector 46 via line 160.

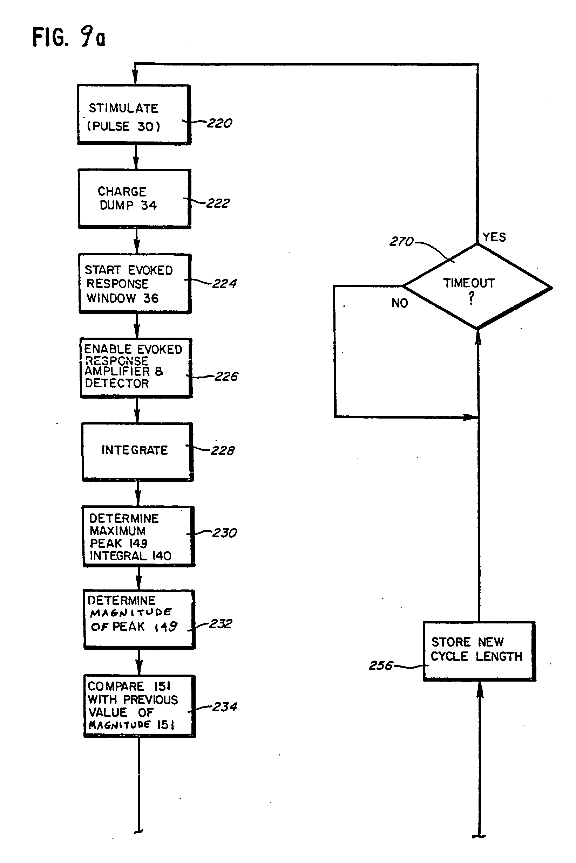

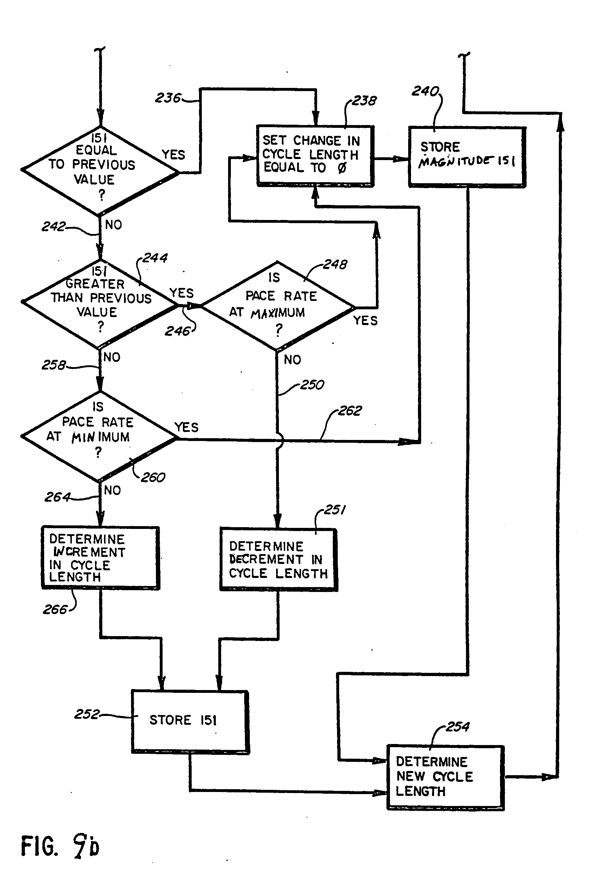

A relatively detailed schematic diagram of the pacer electronics is presented as FIGURE 7. Referring to FIGURE 7, it is seen that the same reference numerals are used for the same components of FIGURE 3. Timing and control circuit 50 comprises a microcomputer 190 which addresses a memory 192 via address bus 194. Data bus 196 is coupled between microcomputer 190 and memory 192, and conventional control logic 198 is coupled to data bus 196. A crystal controlled clock 200 is used for providing appropriate clock pulses for the system. The functions of the control logic inputs and outputs are designated.

Control logic circuit 198 provides a gradient measure enable signal to electronic switch 202 and to analog to digital converter 204 which is at the output of an integrating amplifier 206. It can be seen that the output of the amplifier potential sensed at ring 18 is applied to the negative input of integrating amplifier 206 which, when enabled, provides an amplified analog output that is converted to digital data by means of analog to digital converter 204. The digital data contains the ventricular gradient information, which is provided to the control logic circuit 198 whereby appropriate timing of the stimulation pulses is achieved in response thereto.

The gradient measure enable signal 210 is illustrated in channel G on FIGURE 2. It commences at the same time that the capture detection window 36 commences and the gradient measure enable signal 210 continues for several hundred milliseconds.

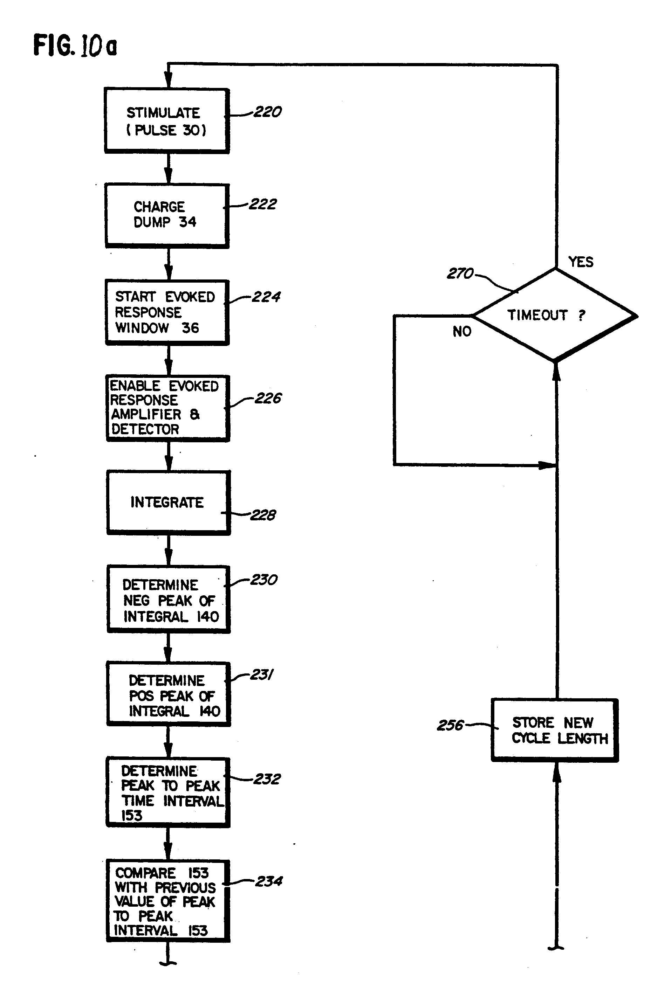

A flow chart illustrating the operation of the first embodiment is presented as Figures 8A-8B. Referring to Figures 8A-8B , the cycle starts with a stimulation pulse 30 being emitted (220). After charge dump 34 (222), the evoked response window 36 commences (224) and the evoked response amplifier and detector is enabled (226). The gradient measure enable signal 210 also commences and the evoked response is integrated (228) and the maximum peak 148 of the integral 140 is determined (230). The magnitude of peak 148, which magnitude is the depolarization gradient magnitude 162, is then determined (232). The depolarization gradient magnitude 162 is then compared (234) with the previous value of the depolarization gradient magnitude 162. If they are equal (236), the pacing rate, i.e., cycle length, is not changed (238) and the magnitude 162 is stored (240). If they are not equal (242), a determination is made (244) whether the present magnitude 162 is greater than the previous magnitude 162. If it is greater (246), a determination (248) is made whether the pacing rate is at its minimum. If it is at its minimum, there will be no further change in pacing rate (238) and the magnitude 162 will be stored (240). If it is not at its minimum (250), a determination (251) will be made with respect to decreasing the present pacing rate, and the magnitude 162 is stored (252). A new decrease pacing rate (i.e., increased cycle length) is determined (254) and stored (256 ).

On the other hand, if it is determined that the present magnitude 162 is not equal to its previous value (242) and is also not greater than its previous value (258), this indicates that present magnitude 162 is less than previous magnitude 162. A determination is then made whether the pacing rate is at its maximum (260) and if it is at its maximum (262), the cycle length is not changed (238) and the magnitude 162 is stored (240). If the pacing rate is not at its maximum (264), a determination (266) is made with respect to the decrement in the cycle length (i.e., the increase in pacing rate) and the magnitude 162 is stored (252). A determination (254) is then made with respect to the new increased pacing rate (i.e., decreased cycle length) and this information is stored.

When the rate timer times out (270), a new stimulation pulse 30 is issued (220) and the cycle will again commence.

A flow chart illustrating the operation of the second embodiment is presented as Figures 9A-9B. Referring to Figures 9A-9B , the cycle starts with a stimulation pulse 30 being emitted (220). After charge dump34 (222), the evoked response window 36 commences (224) and the evoked response amplifier and detector is enabled (226). The gradient measure enable signal 210 also commences and the evoked response is integrated (228) and the maximum peak 149 of the integral 140 is determined (230). The magnitude of peak 149 (from baseline 154 to point 149 in FIGURE 6), which magnitude is the repolarization gradient magnitude 151, is then determined (232). The repolarization gradient magnitude 151 is then compared (234) with the previous value of the repolarization gradient magnitude 151. If they are equal (236), the pacing rate, i.e., cycle length, is not changed (238) and the magnitude 151 is stored (240). If they are not equal (242), a determination is made (244) whether the present magnitude 151 is greater than the previous magnitude 151. If it is greater (246), a determination (248) is made whether the pacing rate is at its maximum. If it is at its maximum, there will be no further change in pacing rate (238) and the magnitude 151 will be stored (240). If it is not at its maximum (250), a determination (251) will be made with respect to increasing the present pacing rate, and the magnitude 151 is stored (252). A new increased pacing rate (i.e., decreased cycle length) is determined (254) and stored (256).

On the other hand, if it is determined that the present magnitude 151 is not equal to its previous value (242) and is also not greater than its previous value (258), this indicates that present magnitude 151 is less than previous magnitude 151. A determination is then made whether the pacing rate is at its minimum (260) and if it is at its minimum (262), the cycle length is not changed (238) and the magnitude 151 is stored (240). If the pacing rate is not at its minimum (264), a determination (266) is made with respect to the increase in the cycle length (i.e., the decrease in pacing rate) and the magnitude 151 is stored (252). A determination (254) is then made with respect to the new decreased pacing rate (i.e., increased cycle length) and this information is stored.

When the rate timer times out (270), a new stimulation pulse 30 is issued (220) and the cycle will again commence.

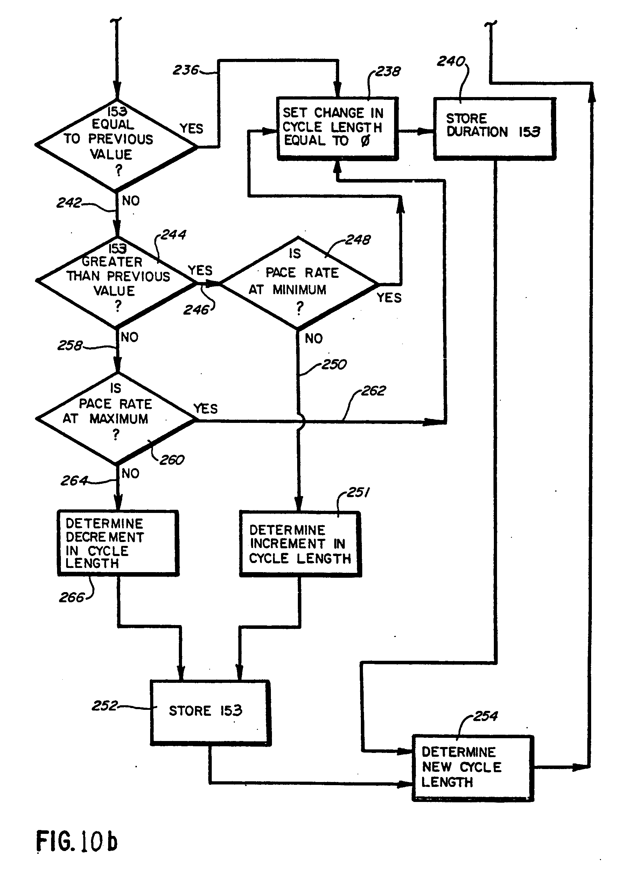

A flow chart illustrating the operation of the third embodiment is presented as Figures 10A-10B. Referring to Figures 10A-10B , the cycle starts with a stimulation pulse 30 being emitted (220). After charge dump34 (222), the evoked response window 36 commences (224) and the evoked response amplifier and detector is enabled (226). The gradient measure enable signal 210 also commences and the evoked response is integrated (228). The negative peak 148 of the integral 140 is determined (230). The positive peak 149 of integral 140 is then determined (231). The time interval 153 between peak 148 and peak 149 is then determined (232). The peak to peak time interval 153 is then compared (234) with the previous peak to peak time interval 153. If they are equal (236), the pacing rate, i.e., cycle length, is not changed (238) and the peak to peak time interval 153 is stored (240). If they are not equal (242), a determination is made (244) whether the peak to peak time interval 153 is greater than the previous peak to peak time interval 153. If it is greater (246), a determination (248) is made whether the pacing rate is at its minimum. If it is at its minimum, there will be no further change in pacing rate (238) and the peak to peak time interval 153 will be stored (240). If it is not at its minimum (250), a determination (251) will be made with respect to decreasing the present pacing rate, and the peak to peak time interval 153 is stored (252). A new decreased pacing rate (i.e., increased cycle length) is determined (254) and stored (256).

On the other hand, if it is determined that the present peak to peak time interval 153 is not equal to its previous value (242) and is also not greater than its previous value (258), this indicates that the present peak to peak time interval 153 is less than the previous peak to peak time interval 153. A determination is then made whether the pacing rate is at its maximum (260) and if it is at its maximum (262), the cycle length is not changed (238) and the peak to peak time interval 153 is stored (240). If the pacing rate is not at its maximum (264), a determination (266) is made with respect to the decrement in the cycle length (i.e., the increase in pacing rate) and the peak to peak time interval 153 is stored (252). A determination (254) is then made with respect to the new increased pacing rate (i.e., decreased cycle length) and this information is stored.

When the rate timer times out (270), a new stimulation pulse 30 is issued (220) and the cycle will again commence.

## Claims

1. Cardiac pacing apparatus which comprises:
means (16, 18) for applying electrical stimulus pulses (30) to the heart (21);
means (46, 54) for detecting a cardiac event potential (38);
means (57) for integrating the detected potential (38) during at least one cardiac cycle to obtain the depolarization gradient magnitude (162) as a selected parameter;
means (192) for storing said selected parameter;
means (190) for comparing said selected parameter with a corresponding selected parameter of at least one previous cardiac cycle that has been stored; and
means (54, 190, 198) for controlling the rate of said electrical stimulus pulses (30) in response to said comparison.

2. Cardiac pacing apparatus as described in claim 1, in which said cardiac event potential (38) comprises a natural ventricular event potential.

3. Cardiac pacing apparatus as described in claim 1, including means for detecting a peak (148) of said integrated cardiac event potential from a base line (154).

4. Cardiac pacing apparatus as described in claim 1, including means (46, 48, 50) for inhibiting the application of a stimulus pulse (30) if a natural ventricular event is sensed within a predetermined time period.

5. Cardiac pacing apparatus as described in claim 1, in which said controlling means (54, 190, 198) includes means (190, 198) for maintaining the pacing rate constant if the compared selected parameters are equal, means for varying the pacing rate in one direction if the selected parameter is less than the prior value, and means for varying the pacing rate in the opposite direction if the selected parameter is greater than the prior value.

6. Cardiac pacing apparatus as described in claim 5, including means for determining if the pacing rate is at a predetermined minimum, means for determining if the pacing rate is at a predetermined maximum, said controlling means being operable to maintain the pacing rate if the pacing rate is at one of a predetermined maximum and a predetermined minimum.

7. Cardiac pacing apparatus as described in claim 1, in which the rate controlling step comprises the following steps:
if the depolarization gradient magnitude (162) is less than the prior value, then determining if the pacing rate is at a predetermined maximum; and if the pacing rate is at a predetermined maximum, then not changing the pacing rate, but if the pacing rate is less than said predetermined maximum then increasing the pacing rate; and if the depolarization gradient magnitude (162) is greater than the prior value, then determining whether the pacing rate is at a predetermined minimum value; and if the pacing rate is a predetermined minimum value, then not changing the pacing rate, but if the pacing rate is greater than said predetermined minimum then decreasing the pacing rate.

8. Cardiac pacing apparatus as described in claim 1, including means (190, 198) for comparing said selected parameter with a target value.

9. Cardiac pacing apparatus as described in claim 8, in which said controlling means (48, 190, 198) includes means for maintaining the pacing rate constant if the compared values are equal, means for varying the pacing rate in one direction if the selected parameter is less than the target value, and means for varying the pacing rate in the opposite direction if the selected parameter is greater than the target value.

10. Cardiac pacing apparatus as described in claim 1, including means (57) for integrating the detected potential over time to obtain the depolarization gradient (141) and a repolarization gradient (147) as a further selected parameter;
means for sensing the peak of the depolarization gradient 141);
means for sensing the peak of the repolarization gradient (147);
means for determining the peak to peak time interval (153) from the depolarization gradient peak (148) to the repolarization gradient peak (149);
means (192) for storing said peak to peak time interval (153);
means (190, 198) for comparing said peak to peak time interval (153) with a corresponding peak to peak time interval of at least one previous cardiac cycle that has been stored; and
means (48, 50, 190, 198) for controlling the rate of said electrical stimulus pulses (30) in response to said comparison.

11. Cardiac pacing apparatus as described in claim 10, including means (190, 198) for comparing said peak to peak time interval (153) with a target value and means (48, 50, 190, 198) for controlling the rate of said electrical stimulus pulses (30) in response to said comparison.

12. Cardiac pacing apparatus as described in claim 11, in which the rate controlling step includes the steps of not changing the pacing rate if the compared peak to peak time interval (153) and target value are equal, varying the pacing rate in one direction if the peak to peak time interval (153) is less than the target value, and varying the pacing rate in the opposite direction if the peak to peak time interval (153) is greater than the target value.

13. Cardiac pacing apparatus as described in claim 12, in which the controlling step includes the steps of not changing the pacing rate if the compared values are equal, increasing the pacing rate if the time interval is less than the target value, and decreasing the pacing rate if the time interval is greater than the target value.

14. Cardiac pacing apparatus which comprises:
means (16, 18, 48)for applying electrical stimulus pulses (30) to the heart (21);
means (46, 54) for detecting a cardiac event potential (38);
means (57) for integrating the detected potential during at least one cardiac cycle to obtain the repolarization gradient (147) as a selected parameter;
means for sensing the peak (149) of said repolarization gradient (147);
means for determining the repolarization gradient magnitude (151);
means (192) for storing said repolarization gradient magnitude;
means (190, 198) for comparing said repolarization gradient magnitude (151) with a corresponding selected parameter of at least one previous cardiac cycle that has been stored; and
means (48, 50, 190, 198) for controlling the rate of said electrical stimulus pulses (30) in response to said comparison.

15. Cardiac pacing apparatus as described in claim 14, in which said target value is a corresponding repolarization gradient magnitude (151) of at least one previous cardiac cycle that has been stored.

## Patentansprüche

1. Herzschrittmacher mit:
Mitteln (16, 18) zum Anlegen eines elektrischen Stimuluspulses (30) an das Herz (21);
Mitteln (46, 54) zum Erfassen eines Herzereignispotentiales (38);
Mitteln (57) zum Integrieren des erfaßten Potentiales (28) während wenigstens eines Herzzyklus zum Erhalten der Depolarisationsgradientgröße (162) als einem ausgewählten Parameter;
Mitteln (192) zum Speichern des ausgewählten Parameters;
Mitteln (190) zum Vergleichen des ausgewählten Parameters mit einem entsprechenden ausgewählten Parameter wenigstens eines vorhergehenden Herzzyklus, welcher gespeichert wurde; und
Mitteln (54, 190, 198) zum Steuern der Rate des elektrischen Stimuluspulses (30) als Antwort auf den Vergleich.

2. Herzschrittmacher nach Anspruch 1, in welchem das Herzereignispotential (38) ein natürliches ventrikuläres Ereignispotential umfaßt.

3. Herzschrittmacher nach Anspruch 1, Mittel einschließend zum Erfassen eines Gipfels (148) des integrierten Herzereignispotentials von einer Basislinie (154).

4. Herzschrittmacher nach Anspruch 1, Mittel (46, 48, 50) einschließend zum Hemmen des Anlegens eines Stimuluspulses (30), wenn ein natürliches ventrikuläres Ereignis innerhalb einer vorbestimmten Zeitperiode erfaßt wird.

5. Herzschrittmacher nach Anspruch 1, in welchem die Steuermittel (54, 190, 198) Mittel (190, 198) einschließen zum Konstanthalten der Schrittmacherrate, wenn die verglichenen ausgwählten Parameter gleich sind, Mittel zum Verändern der Schrittmacherrate in eine Richtung, wenn der ausgewählte Parameter kleiner ist als der vorhergehende Wert, und Mittel zum Variieren der Schrittmacherrate in die entgegengesetzte Richtung, wenn der ausgewählte Parameter größer ist als der vorangehende Wert.

6. Herzschrittmacher nach Anspruch 5, einschließend Mittel zum Bestimmen, wenn die Schrittmacherrate bei einem vorbestimmten Minimum liegt, Mittel zum Bestimmen, wenn die Schrittmacherrate bei einem vorbestimmten Maximum liegt, wobei die Steuermittel betrieben werden können, um die Schrittmacherrate aufrechtzuerhalten, wenn die Schrittmacherrate bei einem von einem vorbestimmten Maximum und einem vorbestimmten Minimum liegt.

7. Schrittmacher nach Anspruch 1, in welchem der Schritt zum Steuern der Rate die folgenden Schritte umfaßt:
wenn die Depolarisationsgradientgröße (162) kleiner ist als der vorangehende Wert, dann bestimmen, wenn die Schrittmacherrate bei einem vorbestimmten Maximum liegt; und wenn die Schrittmacherrate bei einem vorbestimmten Maximum liegt, dann kein Ändern der Schrittmacherrate aber, wenn die Schrittmacherrate kleiner ist als das vorbestimmte Maximum, dann Erhöhen der Schrittmacherrate; und wenn die Depolarisationsgradientgröße (162) größer ist als der vorangehende Wert, dann bestimmen, ob die Schrittmacherrate bei einem vorbestimmten Minimalwert liegt; und, wenn die Schrittmacherrate bei einem vorbestimmten Minimalwert liegt, dann kein Ändern der Schrittmacherrate aber, wenn die Schrittmacherrate größer ist als das vorbestimmte Minimum, dann Verringern der Schrittmacherrate.

8. Herzschrittmacher nach Anspruch 1, Mittel (190, 198) einschließend zum Vergleichen der ausgewählten Parameter mit einem Zielwert.

9. Herzschrittmacher nach Anspruch 8, in welchem die Steuermittel (48, 190, 198) Mittel einschließen zum Konstanthalten der Schrittmacherrate, wenn die verglichenen Werte gleich sind, Mittel zum Ändern der Schrittmacherrate in eine Richtung, wenn der ausgewählte Parameter kleiner ist als der Zielwert und Mittel zum Ändern der Schrittmacherrate in die entgegengesetzte Richtung, wenn der ausgewählte Parameter größer ist als der Zielwert.

10. Herzschrittmacher nach Anspruch 1, Mittel (57) einschließend zum Integrieren des erfaßten Potentiales über die Zeit, um den Depolarisationsgradienten (141) und einen Repolarisationsgradienten (147) als einen weiteren ausgewählten Parameter zu erhalten;
Mittel zum Erfassen des Gipfels des Depolarisationsgradienten (141);
Mittel zum Erfassen des Gipfels des Repolarisationsgradienten (147);
Mittel zum Erfassen des Gipfel-zu-Gipfel-Zeitintervalls (153) von dem Depolarisationsgradientgipfel (148) zu dem Repolarisationsgradientengipfel (149);
Mittel (192) zum Speichern des Gipfel-zu-Gipfel-Zeitintervalles (153);
Mittel (190, 198) zum Vergleichen des Gipfel-zu-Gipfel-Zeitintervalls (153) mit einem entsprechenden Gipfel-zu-Gipfel-Zeitintervall wenigstens eines vorhergehenden Herzzyklus, welcher gespeichert worden ist; und
Mittel (48, 50, 190, 198) zum Steuern der Rate des elektrischen Stimuluspulses (30) als Antwort auf den Vergleich.

11. Herzschrittmacher nach Anspruch 10, Mittel (190, 198) einschließend zum Vergleichen des Gipfel-zu-Gipfel-Zeitintervalls (153) mit einem Zielwert und Mittel (48, 50, 190, 198) zum Steuern der Rate des elektrischen Stimuluspulses (30) als Antwort auf den Vergleich.

12. Herzschrittmacher nach Anspruch 11, in welchem der Schritt zum Steuern der Rate die Schritte einschließt: nicht-Ändern der Schrittmacherrate, wenn das verglichene Gipfel-zu-Gipfel-Zeitintervall (153) und der Zielwert gleich sind, Ändern der Schrittmacherrate in eine Richtung, wenn das Gipfel-zu-Gipfel-Zeitintervall (153) kleiner ist als der Zielwert, und Ändern der Schrittmacherrate in die entgegengesetzte Richtung, wenn das Gipfel-zu-Gipfel-Zeitintervall (153) größer ist als der Zielwert.

13. Herzschrittmacher nach Anspruch 12, in welchem der Steuerschritt die Schritte einschließt: nicht-Ändern der Schrittmacherrate, wenn die verglichenen Werte gleich sind, Erhöhen der Schrittmacherrate, wenn das Zeitintervall kleiner ist als der Zielwert und Verringern der Schrittmacherrate, wenn das Zeitintervall größer ist als der Zielwert.

14. Herzschrittmacher, welcher umfaßt:
Mittel (16, 18, 48) zum Anlegen eines elektrischen Stimuluspulses (30) an das Herz (21);
Mittel (46, 54) zum Erfassen eines Herzereignispotentiales (38);
Mittel (57) zum Integrieren des erfaßten Potentiales während wenigstens eines Herzzyklus', um den Repolarisationsgradienten (147) als einen ausgewählten Parameter zu erhalten;
Mittel zum Erfassen des Gipfels (149) des Repolarisationsgradienten (147);
Mittel zum Bestimmen der Repolarisationsgradientengröße (151);
Mittel (192) zum Speichern der Repolarisationsgradientgröße;
Mittel (190, 198) zum Vergleichen der Repolarisationsgradientgröße (151) mit einem entsprechenden ausgewählten Parameter wenigstens eines vorangehenden Herzzyklus', welcher gespeichert worden ist; und
Mittel (48, 50, 190, 198) zum Steuern der Rate des elektrischen Stimuluspulses (30) als Antwort auf den Vergleich.

15. Herzschrittmacher nach Anspruch 14, in welchem der Zielwert eine entsprechende Repolarisationsgradientgröße (151) wenigstens eines vorangehenden Herzzyklus ist, welcher gespeichert worden ist.

## Revendications

1. Appareil de stimulation cardiaque comprenant:
des moyens (16, 18) pour appliquer des impulsions de stimulus électrique (30) au coeur (21);
des moyens (46, 54) pour détecter un potentiel d'événement cardiaque (38);
des moyens (57) pour intégrer le potentiel détecté (38), pendant au moins un cycle cardiaque, afin d'obtenir l'amplitude de gradient de dépolarisation (162), à titre de paramètre sélectionné;
des moyens (192) pour enregistrer le paramètre sélectionné;
des moyens (190) pour comparer le paramètre sélectionné avec un paramètre sélectionné correspondant d'au moins un cycle cardiaque précédent qui a été enregistré; et
des moyens (54, 190, 198) pour commander la cadence des impulsions de stimulus électrique (30) sous la dépendance de la comparaison précitée.

2. Appareil de stimulation cardiaque décrit dans la revendication 1, dans lequel le potentiel d'événement cardiaque (38) consiste en un potentiel d'événement venticulaire naturel.

3. Appareil de stimulation cardiaque décrit dans la revendication 1, comprenant des moyens pour détecter une crête (148) du potentiel d'événement cardiaque intégré, à partir d'une ligne de base (154).

4. Appareil de stimulation cardiaque décrit dans la revendication 1, comprenant des moyens (46, 48, 50) pour empêcher l'application d'une impulsion de stimulation (30) si un événement ventriculaire naturel est détecté pendant un intervalle de temps prédéterminé.

5. Appareil de stimulation cardiaque décrit dans la revendication 1, dans lequel les moyens de commande (54, 190, 198) comprennent des moyens (190, 198) pour maintenir la cadence de stimulation à une valeur constante si les paramètres sélectionnés qui sont comparés sont égaux, des moyens pour faire varier la cadence de stimulation dans un sens si le paramètre sélectionné est inférieur à la valeur antérieure, et des moyens pour faire varier la cadence de stimulation dans le sens opposé si le paramètre sélectionné est supérieur à la valeur antérieure.

6. Appareil de stimulation cardiaque décrit dans la revendication 5, comprenant des moyens pour déterminer si la cadence de stimulation est à un minimum prédéterminé, et des moyens pour déterminer si la cadence de stimulation est à un maximum prédéterminé, les moyens de commande étant capables de maintenir la cadence de stimulation si cette dernière est soit à un maximum prédéterminé, soit à un minimum prédéterminé.

7. Appareil de stimulation cardiaque décrit dans la revendication 1, dans lequel l'étape de commande de la cadence comprend les étapes suivantes :
si l'amplitude de gradient de dépolarisation (162) est inférieure à la valeur antérieure, on détermine si la cadence de stimulation est à un maximum prédéterminé; et si la cadence de stimulation est à un maximum prédéterminé, on ne change pas la cadence de stimulation, mais si la cadence de stimulation est inférieure au maximum prédéterminé, on augmente la cadence de stimulation; et si l'amplitude de gradient de dépolarisation (162) est supérieure à la valeur antérieure, on détermine si la cadence de stimulation est à une valeur minimale prédéterminée, et si la cadence de stimulation est à une valeur minimale prédéterminée, on ne change pas la cadence de stimulation, mais si la cadence de stimulation est supérieure à la valeur minimale prédéterminée, on diminue la cadence de stimulation.

8. Appareil de stimulation cardiaque décrit dans la revendication 1, comprenant des moyens (190, 198) pour comparer le paramètre sélectionné avec une valeur de consigne.

9. Appareil de stimulation cardiaque décrit dans la revendication 8, dans lequel les moyens de commande (48, 190, 198) comprennent des moyens pour maintenir la cadence de stimulation constante si les valeurs comparées sont égales, des moyens pour faire varier la cadence de stimulation dans un sens si le paramètre sélectionné est inférieur à la valeur de consigne, et des moyens pour faire varier la cadence de stimulation dans le sens opposé si le paramètre sélectionné est supérieur à la valeur de consigne.

10. Appareil de stimulation cardiaque décrit dans la revendication 1, comprenant des moyens (57) pour intégrer au cours du temps le potentiel détecté, afin d'obtenir un gradient de dépolarisation (141) et un gradient de repolarisation (147), à titre de paramètre sélectionné supplémentaire;
des moyens pour détecter la crête du gradient de dépolarisation (141);
des moyens pour détecter la crête du gradient de repolarisation (147);
des moyens pour détecter l'intervalle de temps crête à crête (153) de la crête du gradient de dépolarisation (148) jusqu'à la crête du gradient de repolarisation (149);
des moyens (192) pour enregistrer l'intervalle de temps crête à crête (153);
des moyens (190, 198) pour comparer l'intervalle de temps crête à crête (153) avec un intervalle de temps crête à crête correspondant d'au moins un cycle cardiaque précédent qui a été enregistré; et
des moyens (48, 50, 190, 198) pour commander la cadence des impulsions de stimulus électrique (30) sous la dépendance de la comparaison précitée.

11. Appareil de stimulation cardiaque décrit dans la revendication 10, comprenant des moyens (190, 198) pour comparer l'intervalle de temps crête à crête (153) avec une valeur de consigne, et des moyens (48, 50, 190, 198) pour commander la cadence des impulsions de stimulation électrique (30) sous la dépendance de la comparaison précitée.

12. Appareil de stimulation cardiaque décrit dans la revendication 11, dans lequel l'étape de commande de cadence comprend les étapes suivantes : on ne change pas la cadence de stimulation si l'intervalle de temps crête à crête (153) et la valeur de consigne qui sont comparées sont égaux, on fait varier la cadence de stimulation dans un sens si l'intervalle de temps crête à crête (153) est inférieur à la valeur de consigne, et on fait varier la cadence de stimulation dans le sens opposé si l'intervalle de temps crête à crête (153) est supérieur à la valeur de consigne.

13. Appareil de stimulation cardiaque décrit dans la revendication 12, dans lequel l'étape de commande comprend les étapes suivantes : on ne change pas la cadence de stimulation si les valeurs comparées sont égales, on augmente la cadence de stimulation si l'intervalle de temps est inférieur à la valeur de consigne, et on diminue la cadence de stimulation si l'intervalle de temps est supérieur à la valeur de consigne.

14. Appareil de stimulation cardiaque comprenant :
des moyens (16, 18, 48) pour appliquer des impulsions de stimulation électrique (30) au coeur (21);
des moyens (46, 54) pour détecter un potentiel d'événement cardiaque (38);
des moyens (57) pour intégrer le potentiel détecté pendant au moins un cycle cardiaque, afin d'obtenir le gradient de repolarisation (147), à titre de paramètre sélectionné;
des moyens pour détecter la valeur de crête (149) du gradient de repolarisation (147);
des moyens pour déterminer l'amplitude de gradient de repolarisation (151);
des moyens (192) pour enregistrer l'amplitude de gradient de repolarisation;
des moyens (190, 198) pour comparer l'amplitude de gradient de repolarisation (151) avec un paramètre sélectionné correspondant d'au moins un cycle cardiaque précédent qui a été enregistré; et
des moyens (48, 50, 190, 198) pour commander la cadence des impulsions de stimulation électrique (30) sous la dépendance de la comparaison précitée.

15. Appareil de stimulation cardiaque décrit dans la revendication 14, dans lequel la valeur de consigne est une amplitude de gradient de repolarisation (151) d'au moins un cycle cardiaque précédent, qui a été enregistrée.
